# EUROPEAN PATENT SPECIFICATION

(11) **EP 0 786 828 B1**
(45) Date of publication and mention of the grant of the patent: **08.12.1999**
(21) Application number: 96115777.3
(22) Date of filing: 02.10.1996
(51) Int. Cl.: H01R 4/48, H01R 4/22, A61N 1/05, H01R 4/26

(54) **A method of forming an electrical connection device**
Verfahren zur Herstellung eines Elektrischen Verbindungsgeraets
Méthode pour réaliser un dispositif de raccordement électrique

(30) Priority: 06.10.1995 IT TO950801
(43) Date of publication of application: 30.07.1997
(73) Proprietor: SORIN BIOMEDICA CARDIO S.p.A., 13040 Saluggia (Vercelli) (IT)
(72) Inventor: Cerise, Osvaldo, 11100 Aosta (IT)
(74) Representative: Bosotti, Luciano

(56) References cited:
- GB-A- 1 519 769
- US-A- 3 437 091
- US-A- 4 530 560
- US-A- 5 433 734

## Description

The present invention relates to electrical connection devices and has been developed with particular attention to its possible use in the biomedical field.

In this field of use, a problem often arises in making electrical connections from and to wires of very small cross-section, such as those used, for example, for transferring sensing and/or stimulation signals in electro-catheters. These electric wires are often covered by insulating sheaths so that, when the connection is made, it is also necessary to expose the wire locally by stripping it of its sheath.

The solutions most generally used in this field usually provide for the wire, previously stripped and possibly wound in a helix, to be inserted and compressed between two bush elements, of which one, normally that positioned on the outside, is deformed plastically (a so-called "crimping" operation) so as to bring about a stable contact configuration. A substantially similar arrangement is disclosed, e.g. in US-A-5 433 734, after which the preamble of Claim 1 was patterned.

Although widely practised, this solution is not without problems, such as the need, in the case of covered wires, for the wire to be stripped beforehand, which operation may not be easy to carry out when the wire has to be wound in a helix in the contact region and/or when the contact has to be made in a position to which access is difficult, for example, because it is in a region in which many electrical connections have to be made very close to one another.

The object of the present invention is to provide a solution which can solve the problems set out above in a simple and reliable manner.

According to the present invention, this object is achieved by means of a method of forming an electrical connection device having the specific characteristics claimed in Claim 1.

The invention will now be described, purely by way of non-limiting example, with reference to the appended drawings, in which:
Figures 1 and 2 show schematically, in axial section, the structure of an electrical connection device formed according to the invention in two successive possible operating conditions, and
Figure 3 shows, in detail, a practical example of the use of a connection device according to the invention in an electro-catheter.

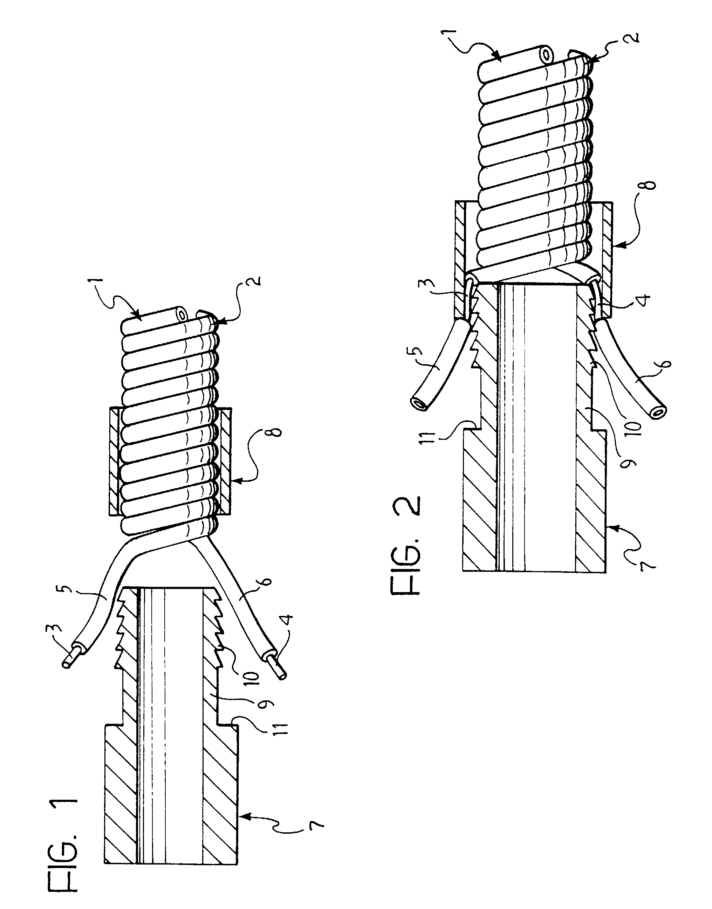

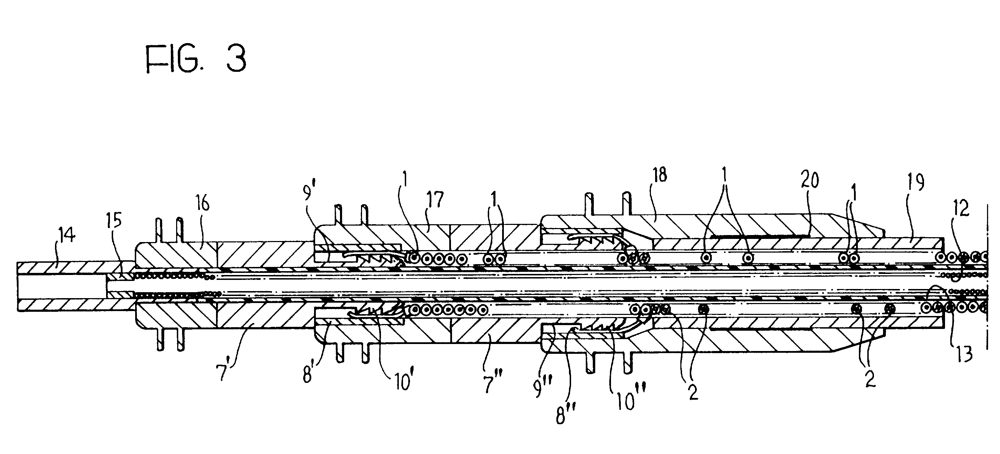

In Figures 1 and 2, two electric wires, indicated 1 and 2 respectively, are wound in helices with interlaced turns (that is, with an alternating sequence of turns belonging to one wire and to the other, respectively). For example, as can best be seen with reference to Figure 3, these may be the two electric wires included in an electro-catheter.

The wires are therefore very small, for example, comprising at least one conductive centre or core 3, 4 of copper, aluminium, silver, etc, with a circular cross-section and a diameter, for example, of the order of 0.1-0.5 mm, covered by a corresponding sheath 5, 6 made of electrically insulating material and also circular.

It should, however, be pointed out that Figures 1 and 2 relate to the presence of two wires 1, 2 wound in interlaced helices as described above, purely by way of example.

As will be explained further below, and also with reference to the specific example of application of Figure 3, the solution according to the invention can be used in association with wires wound in helices with turns interlaced in a different manner, for example, with pairs of turns belonging to one wire interposed between pairs of turns belonging to another wire. Moreover, the wire connected may be a single wire or a multifilamentary wire with any number n of cores. Furthermore, the wire or wires connected do not necessarily have to be arranged in a helix and may very well be wires extending along any path, usually a straight path.

The connection device according to the invention comprises essentially two complementary sleeve elements, a first (male) element and a second (female) element, indicated 7 and 8, respectively.

As will be understood better from the following, the general reference to a sleeve-like configuration is made purely by way of example. In fact, at least one of the elements in question, for example, the male element 7, could very well be in the form of a solid body.

In the embodiment shown, the element indicated 7 is formed as a kind of plug or pin comprising an approximately tubular base body from which a further tubular element 9 with a diameter slightly smaller than that of the base portion extends as the actual male element (the coupling portion). The coupling portion 9 has - at its free end which may be defined as the distal end - a series of circular, peripheral ribs (usually continuous ribs, but theoretically also discontinuous ribs), indicated 10, and having general cutting profiles, shown here as sawtooth profiles, with the steep side of the profile of each rib facing towards the base portion. Consequently, the less steep side of the profile of the ribs 10 faces towards the distal end of the male element 7 and hence, during coupling, towards the female element, that is, the coupling element other than the one on which the ribs are disposed.

In the following description, reference will be made, at least implicitly, to elements 7 and 8 having circular cross-sections; however, although it is preferred, at least for reasons of simplicity, this characteristic is not essential; the elements 7 and 8 may in fact have any cross-sectional profile, for example, a polygonal or ellipsoidal profile, etc.

The female element 8 is constituted essentially by a sleeve, the cavity or axial opening of which has (inside) diametral dimensions selected so as to correspond approximately to the outside diametral dimensions of the tip portions, and hence of the cutting portions, of the sawtooth-shaped ribs 10 of the male element.

The term "correspond approximately" should be interpreted in the sense that, in the undeformed condition, the inside diameter selected for the sleeve element 8 (for example, of the order of 1-2 cm) is substantially equal to or slightly greater than the outside diameters of the ribs 10 on the distal portion of the male element 7, account being taken of the diameters of the wires 1 and 2.

It is desirable, preferably, to arrange for the sleeve-like portion constituting the female element 8 to undergo a certain deformation in the sense that it becomes more oval during coupling with the portion 9 (as will be described further below).

This result can easily be achieved if the female element 8 (and also, preferably, the male element 7) is made of a conductive material such as a metallic material, for example stainless steel, titanium, or Pt-Ir.

The sequence of Figures 1 and 2 shows schematically how the electrical connection device constituted by the elements 7 and 8 is assembled.

In practice, in order to make the connection, the wire or wires 1, 2 are inserted in the female element 8, with their free ends which are to abut the connection device extending in a generally axial position relative to the female element 8 and to the male element which has previously been brought close to the female element, as shown schematically in Figure 1.

In particular, the wire or wires 1, 2 are arranged so that their free ends bear on the ribs 10 approximately axially relative to the portion 9 of the male element 7.

At this point, the elements 7, 8 are moved towards one another, as shown schematically in Figure 2, so that the female element 8 is made to slide on the portion 9 of the male element 7, particularly on the ribs 10.

On account of the dimensional relationship existing between the outside diameters of the ribs 10, the inside diameter of the female element 8, and the diameter of the wire or wires 1, 2, the latter are squeezed between the female element 8, which is usually slightly resiliently deformed, and the ribs 10, particularly the cutting edge of each rib. The effect of this cutting edge is such as to cut the sheath 5, 6 of each wire 1, 2, with the result that the sheath 5, 6 is stripped from the rest of the wire 1, 2 and removed and cleared away from the connection region as a result of the gradual advance of the female element 8 relative to the male element 7. The conductive cores 3, 4 are thus exposed.

As stated, during the aforementioned advancing movement, the female element 8 is deformed resiliently owing precisely to the reaction exerted by the stripped portion of wire (the centre or core) disposed between the internal surface of the female element 8 and the outer peripheries of the ribs 10.

In the specific embodiment illustrated, in which two wires 1, 2 are diametrally opposed relative to the male element 7, this resilient deformation of the female element 8 is brought about essentially in the sense that it becomes more oval. More generally, this deformation may be seen as a deformation which causes the female element 8, which usually has a circular initial profile, to adopt a lobed configuration with one or more lobes, each corresponding to the angular position, relative to the ribs 10, adopted by the wire or wires 1, 2 being connected.

The advancing movement of the female element 8 relative to the male element 7 in practice continues until the female element 8 has passed over the entire axial section of the portion 9 of the male element 7 on which the sawtooth-shaped ribs 10 are provided. In practice, this corresponds to the abutment of the female element 8 with the shoulder, indicated 11 in Figures 1 and 2, which separates the base portion of the male element 7 from the narrower portion 9 on which the sawtooth-shaped ribs 10 are provided.

The final configuration adopted by the connection device is essentially that shown in Figure 3, in which parts identical or functionally equivalent to those described with reference to Figures 1 and 2 are indicated by the same reference numerals already used above. In particular, with reference to an application in which the basic structure of the invention is duplicated, the elements indicated by the reference numerals 7, 8, 9 and 10 in Figures 1 and 2 have been reproduced once as 7', 8', 9' and 10' and for a second time as 7'', 8'', 9'' and 10''.

Figure 3, which relates to the proximal end of an electro-catheter, shows specifically how the solution according to the invention can advantageously coexist with conventional solutions for making electrical connections, particularly in electrical wires of small dimensions.

In the specific embodiment of Figure 3, the electro-catheter shown comprises, as the central electrode, (typically a stimulation electrode) a helical element 12 constituted by a bare wire (that is, without an insulating covering per se) surrounded by an insulating sheath 13 which surrounds the helix of the wire 12 and is connected, towards the proximal end of the electro-catheter, to a central terminal 14 of conductive material, typically metal. The terminal 14 is tubular and the helical conductor 12 is enveloped so as to be clamped in situ as a result of a plastic deformation (squashing or so-called crimping) of the material constituting the terminal 14.

Two wires, indicated 1 and 2 (for similarity with Figures 1 and 2), are wound around the protective sheath 13, both wires being constituted by covered wires (thus each with a respective sheath of insulating material) and usually being intended to act as carriers for transmitting detection (sensing) and/or command signals from and towards the distal end of the electro-catheter.

The advantages of the use of the solution according to the invention are clear from an observation that the connection system described in detail in the sequence of Figures 1 and 2 enables the two wires 1 and 2 (which, in the example, are constituted by double wires) to be interconnected, respectively, with the elements 7' and 7'' which are electrically independent of one another and of the central terminal 14, avoiding the preliminary operation to remove the sheath. In fact, the procedure illustrated in the sequence of Figures 1 and 2 enables the wires 1 and 2, and hence each of the two pairs of coils (shown paired in the drawing but each belonging to a different wire), to be stripped and clamped simply as a result of being laid axially on the ribs 10' and 10", respectively, with the female elements 8' and 8'' subsequently being placed on the portions 9' and 9'' of the male elements 7' and 7''.

The electro-catheter can thus be completed by the fitting of an insulating sleeve 16 between the elements 14 and 7', an insulating sleeve 17 between the elements 7' and 7'' and an insulating sleeve 18 the proximal end of which is adjacent the portion of the element 7'' which acts as a shoulder for the female element 8''. The sleeve 18 in turn houses a further tubular sheath 19 which covers the end intended to be inserted into the patient's body, according to known criteria. An identification label, indicated 20, is preferably interposed between the sleeve 18 and the sheath 19 (at least the former element being made, at least locally, of transparent material) for identifying the electro-catheter without the possibility of accidental removal.

Naturally, the principle of the invention remaining the same, the details of construction and forms of embodiment may be varied widely with respect to those described and illustrated, without thereby departing from the scope of the present invention.

In particular, although in the foregoing description of a preferred embodiment of the invention reference has been made to the presence of the ribs 10 on the male element 7, the arrangement could be reversed, with the provision of an externally smooth coupling portion 9 and, on the other hand, the arrangement of the ribs in the internal cavity of the female element 8. A combined embodiment in which ribs such as the ribs 10 are present both on the outer surface of the portion 9 and on the inner surface of the element 8 is also possible.

## Claims

1. A method of forming an electrical connection device for the electrical connection of at least one wire (1, 2) comprising at least one conductive core (3, 4) covered by a respective sheath (5, 6), comprising the steps of :
- providing a first element (7) having a coupling portion (9) with given diametral dimensions, and
- providing a second element (8) having an internal cavity so that the second element (8) can be fitted onto the coupling portion (9) of the first element (7),
characterized in that it comprises the further steps of :
- providing at least one of the first element (7) and the second element (8) with at least one peripheral rib (10) with a cutting profile,
- disposing the at least one wire (1, 2) on the coupling portion (9) of the first element (7)
- fitting the second element (8) onto the coupling portion (9) to force the at least one wire (1) against the at least one rib (10)
- advancing the second element (8) relative to the first element (7) causing the respective sheath (5, 6) to be cut and subsequently removed with the at least one conductive core (3, 4) held between the coupling portion (9) of the first element (7) and the second element (8) fitted thereon.

2. A method according to Claim 1, characterized in that the internal cavity of the second element (8) has diametral dimensions substantially corresponding to the diametral dimensions of the coupling portion (9) of the first element (7).

3. A method according to Claim 1 or Claim 2, characterized in that it includes the step of providing the coupling portion (9) of the first element (7) with a profiled portion adapted to penetrate the second element (8), which has a generally tubular structure.

4. A method according to any one of Claims 1 to 3, characterized in that it includes the step of providing the first element (7) with a tubular structure at least in the coupling portion (9).

5. A method according to any one of the preceding claims, characterized in that it includes the step of providing the at least one peripheral rib (10) with a generally annular shape.

6. A method according to any one of the preceding claims, characterized in that it includes the step of providing the at least one peripheral rib (10) with a profile with first and second sides which are steeper and less steep, respectively, and in that, during said fitting step the less steep side is kept facing towards the one of said first and second elements (7, 8) other than the one of said elements on which the rib (10) is disposed.

7. A method according to any one of the preceding claims, characterized in that it includes the step of providing the at least one rib (10) with a sawtooth-like profile.

8. A method according to any one of the preceding claims, characterized in that it includes the step of providing at least one of the first element (7) and the second element (8) with a plurality of peripheral ribs (10).

9. A method according to any one of the preceding claims, characterized in that it includes the step of providing at least one of the first element (7) and the second element (8) with a shoulder formation (11) constituting an abutment surface for the other of the first element (7) and the second element (8).

10. A method according to any one of the preceding claims, characterized in that it includes the step of providing the second element (8) with a generally annular shape and of resiliently deformable material so that, during the coupling with the first element (7), the second element (8) is deformed so as to have a lobed geometry, with each lobe corresponding to a respective wire (1) held between the coupling portion and the second element.

## Patentansprüche

1. Verfahren zur Herstellung einer elektrischen Verbindungsanordnung zur elektrishen Verbindung mit zumindest einem Draht (1, 2), der zumindest eine leitende Seele (3, 4) aufweist, die von einer entsprechenden Ummantelung (5, 6) umgeben ist, wobei das Verfahren die folgenden Schritte umfaßt:
- Bereitstellung eines ersten Elementes (7), das eine Kupplungsabschnitt (9) mit einem vorgegebenen Durchmesser aufweist, und
- Bereitstellung eines zweiten Elementes (8), das einen inneren Hohlraum aufweist, sodaß das zweite Element (8) auf den Kupplungsabschnitt (9) des ersten Elementes (7) aufgebracht werden kann,
**dadurch gekennzeichnet**, daß das Verfahren die weiteren Schritte umfaßt:
- Anbringung zumindest einer Umfangsrippe (10) mit einem Schneidprofil an zumindest einem der beiden Elemente (7, 8)
- Anordnen mindestens eines Drahtes (1, 2) an dem Kupplungsabschnitt (9) des ersten Elementes (7),
- Aufbringen des zweiten Elementes (8) auf den Kupplungsabschnitt (9), um den mindestens einen Draht (1) gegen die zumindest eine Rippe (10) zu pressen
- Verschieben des zweiten Elementes (8) gegenüber dem ersten Element (7) wodurch die entsprechende Ummantelung (5, 6) geschnitten und nachfolgend abgezogen wird und zumindest eine leitende Seele (3, 4) zwischen dem Kupplungsabschnitt (9) des ersten Elementes (7) und dem auf diesen aufgebrachten zweiten Element (8) gehalten wird.

2. Verfahren nach Anspruch 1, **dadurch gekennzeichnet**, daß der innere Hohlraum des zweiten Elementes (8) eine diametrale Abmessung aufweist, die im wesentlichen der diametralen Abmessung des Kupplungsabschnittes (9) des ersten Elementes (7) entspricht.

3. Verfahren nach Anspruch 1 oder 2, **dadurch gekennzeichnet**, daß es den Schritt der Ausgestaltung eines profilierten Abschnittes auf dem Kupplungsabschnitt (9) des ersten Elementes (7) einschließt, der bestimmt ist um in das zweite Element (8) einzudringen, das im wesentlichen einen rohrförmigen Aufbau aufweist.

4. Verfahren nach einem der Ansprüche 1 bis 3, **dadurch gekennzeichnet**, daß es den Schritt der Ausgestaltung des ersten Elementes (7) zumindest im Bereich des Kupplungsabschnittes (9) in Form eines rohrförmigen Aufbau einschließt.

5. Verfahren nach einem der vorhergehenden Ansprüche, **dadurch gekennzeichnet**, daß es den Schritt der Ausgestaltung der mindestens einen Umfangsrippe (10) mit einer im wesentlichen ringförmigen Gestalt einschließt.

6. Verfahren nach einem der vorhergehenden Ansprüche, **dadurch gekennzeichnet**, daß es den Schritt der Ausgestaltung der mindestens einen Umfangsrippe (10) mit einem Profil mit ersten und zweiten Seiten, die mehr oder weniger steil sind einschließt, und daß während des Schrittes des Aufbringens die weniger steile Seite dem jeweils anderen der ersten und zweiten Elemente (7, 8) zugekehrt ist, als jenem an dem die Rippe (10) angeformt ist.

7. Verfahren nach einem der vorhergehenden Ansprüche, **dadurch gekennzeichnet**, daß es den Schritt der Ausgestaltung der mindestens einen Rippe (10) mit einem sägezahnartigen Profil umfaßt.

8. Verfahren nach einem der vorhergehenden Ansprüche, **dadurch gekennzeichnet**, daß es den Schritt der Ausgestaltung mindestens eines der beiden Elemente (7, 8) mit einer Vielzahl von Umfangsrippen (10) einschließt.

9. Verfahren nach einem der vorhergehenden Ansprüche, **dadurch gekennzeichnet**, daß es den Schritt der Ausgestaltung mindestens eines der beiden Elemente (7, 8) mit einer Schulter (11) einschließt, die eine Anschlagsfläche für des jeweils andere der beiden Elemente (7, 8) bildet.

10. Verfahren nach einem der vorhergehenden Ansprüche, **dadurch gekennzeichnet**, daß es den Schritt der Ausgestaltung des zweiten Elementes (8) mit einer im allgemeinen ringförmigen Gestalt und aus einem elastisch verformbaren Material einschließt, so daß während der Kopplung mit dem ersten Element (7) das zweiten Element (8) verformt wird, daß es eine ausgebauchte Geometrie aufweist, wobei jede Ausbauchung mit einem entsprechenden Draht (1) korrespondiert, der zwischen dem Kupplungabschnitt und dem zweiten Element gehalten ist.

## Revendications

1. Procédé pour fabriquer un dispositif de connexion électrique pour établir la connexion électrique d'au moins un fil (1, 2) comprenant au moins une âme conductrice (3, 4) revêtue d'une gaine respective (5, 6) comprenant les étapes consistant à :
- fournir un premier élément (7) ayant une partie de couplage (9) ayant des dimensions diamétrales données, et
- fournir un second élément (8) ayant une cavité intérieure, de telle sorte que le second élément (8) puisse être ajusté sur la partie de couplage (9) du premier élément (7),
caractérisé en ce qu'il comprend en outre les étapes consistant à :
- munir au moins un élément parmi le premier élément (7) et le second élément (8) d'au moins une nervure périphérique (10) d'un profil de coupe,
- disposer le au moins un fil (1, 2) sur la partie de couplage (9) du premier élément (7),
- ajuster le second élément (8) sur la partie de couplage (9) pour amener de force le au moins un fil (1) contre la au moins une nervure (10),
- faire avancer le second élément (8) par rapport au premier élément (7), ce qui entraîne la coupe et ultérieurement le retrait de la gaine respective (5, 6), la au moins une âme conductrice (3, 4) étant maintenue entre la partie de couplage (9) du premier élément (7) et du second élément (8) ajusté sur celui-ci.

2. Procédé selon la revendication 1, caractérisé en ce que la cavité intérieure du second élément (8) présente des dimensions diamétrales correspondant sensiblement aux dimensions diamétrales de la partie de couplage (9) du premier élément (7).

3. Procédé selon la revendication 1 ou la revendication 2, caractérisé en ce qu'il comprend l'étape consistant à munir la partie de couplage (9) du premier élément (7) d'une partie profilée adaptée pour pénétrer dans le second élément (8), qui présente une structure généralement tubulaire.

4. Procédé selon l'une quelconque des revendications 1 à 3, caractérisé en ce qu'il comprend l'étape consistant à munir le premier élément (7) d'une structure tubulaire au moins dans la partie de couplage (9),

5. Procédé selon l'une quelconque des revendications précédentes, caractérisé en ce qu'il comprend l'étape consistant à conférer à la au moins une nervure périphérique (10) une forme généralement annulaire.

6. Procédé selon l'une quelconque des revendications précédentes, caractérisé en ce qu'il comprend l'étape consistant à conférer à la au moins une nervure périphérique (10) un profil avec des premier et second côtés qui sont respectivement plus raides et moins raides, et en ce que, pendant l'étape d'ajustage, le côté moins raide est maintenu tourné vers l'élément parmi lesdits premier et second éléments (7, 8) autres que l'élément parmi lesdits éléments sur lesquels est disposée la nervure (10).

7. Procédé selon l'une quelconque des revendications précédentes, caractérisé en ce qu'il comprend l'étape consistant à conférer à la au moins une nervure (10) un profil en dents de scie.

8. Procédé selon l'une quelconque des revendications précédentes, caractérisé en ce qu'il comprend l'étape consistant à munir au moins un élément parmi le premier élément (7) et le second élément (8) d'une pluralité de nervures périphériques (10).

9. Procédé selon l'une quelconque des revendications précédentes, caractérisé en ce qu'il comprend l'étape consistant à munir au moins un élément parmi le premier élément (7) et le second élément (8) d'une formation en épaulement (11) constituant une surface de butée pour l'autre élément parmi le premier élément (7) et le second élément (8).

10. Procédé selon l'une quelconque des revendications précédentes, caractérisé en ce qu'il comprend l'étape consistant à conférer au second élément (8) une forme généralement annulaire et en matériau élastiquement déformable de telle sorte que, lors du couplage avec le premier élément (7), le second élément (8) est déformé de manière à avoir une géométrie lobée, chaque lobe correspondant à un fil respectif (1) étant maintenu entre la partie de couplage et le second élément.
